## Europäisches Patentamt

## European Patent Office

## Office européen des brevets

(19)

(11) Veröffentlichungsnummer: **0 096 796**
**B1**

(12)

# EUROPÄISCHE PATENTSCHRIFT

(45) Veröffentlichungstag der Patentschrift:
**13.05.87**

(21) Anmeldenummer: **83105357.4**

(22) Anmeldetag: **31.05.83**

(51) Int. Cl.⁴: **B 01 J 29/06, C 07 C 45/53,**
**C 07 C 49/403, C 07 C 29/48**

(54) Kobalt enthaltende Trägerkatalysatoren, deren Herstellung und Verwendung.

(30) Priorität: **11.06.82 DE 3222143**

(43) Veröffentlichungstag der Anmeldung:
**28.12.83 Patentblatt 83/52**

(45) Bekanntmachung des Hinweises auf die Patenterteilung:
**13.05.87 Patentblatt 87/20**

(84) Benannte Vertragsstaaten:
**BE CH DE FR GB IT LI NL**

(56) Entgegenhaltungen:
**EP - A - 0 027 937**
**DE - A - 1 002 754**
**US - A - 3 013 990**
**US - A - 3 783 123**
**US - A - 4 070 403**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft,**
**Carl-Bosch-Strasse 38, D-6700 Ludwigshafen (DE)**

(72) Erfinder: **Hartig, Juergen, Dr., In der Schleit 9,**
**D-6718 Gruenstadt (DE)**
Erfinder: **Stoessel, Armin, Dr., Immengaertenweg 20,**
**D-6710 Frankenthal (DE)**
Erfinder: **Hermann, Guenter, Dr., Haeuserstrasse 21,**
**D-6900 Heidelberg (DE)**
Erfinder: **Marosi, Laszlo, Dr., Leuschnerstrasse 32,**
**D-6700 Ludwigshafen (DE)**

**Beschreibung**

Gegenstand der Erfindung sind Trägerkatalysatoren mit einem Gehalt von 2 bis 30 Gew.% Kobalt in oxidischer Form, berechnet als Kobalt.

Bei der Oxidation von Cyclohexan mit Luft oder Sauerstoff unter erhöhtem Druck und bei erhöhter Temperatur erhält man Gemische aus Cyclohexanol, Cyclohexanon, Cyclohexylhydroperoxid, Säuren und Estern. Zur Verbesserung der Ausbeute wird üblicherweise das gebildete Hydroperoxid unter Mitverwendung von homogen gelösten Metallsalzen, wie Kobalt- oder Chromsalzen zersetzt, wie aus der DE-C-1 002 754 zu entnehmen ist. Auch Kupfer- und Mangansalze wurden bereits hierfür verwendet, wie aus der DE-B-11 93 501 bekannt ist.

Es wurden auch schon Trägerkatalysatoren für die Zersetzung von Cyclohexylhydroperoxid enthaltenden Gemischen verwendet. In der US-PS-2 851 496 werden Metalle der 8. Gruppe, unter anderem Kobaltoxid, auf aktiviertem Aluminiumoxid, Kohle, Silikatgel oder Kieselgur als geeignete Katalysatoren beschrieben. Auch der Einsatz eines Kobaltkatalysators auf zeolithischem Träger ist bereits bekannt, vgl. US-A-3 783 123. Der Literatur (DE-A-23 52 378) ist jedoch zu entnehmen, dass die Umsatzgeschwindigkeit gering und der Katalysatorbedarf hoch ist.

Ähnliche Mängel ergeben sich bei der Durchführung der Reaktion bei höheren Temperaturen, wie sie aus Kogyo Kagaku Shashi, Band 73 (1970), S. 2056ff, bekannt ist. Durch die höheren Temperaturen wird zwar die Reaktionsgeschwindigkeit erhöht, jedoch die Ausbeute vermindert, durch die bei den genannten Temperaturen ablaufende thermische Zersetzung des Peroxids.

Entsprechend der DE-A-23 52 378 sollen heterogene Chromoxidkatalysatoren für die Zersetzung von Cyclohexylhydroperoxid verwendet werden. Diese Katalysatoren entsprechen jedoch hinsichtlich ihrer Lebensdauer und Wasserempfindlichkeit nicht den technischen Anforderungen.

Es war deshalb die technische Aufgabe gestellt, für die Umwandlung von Cyclohexylhydroperoxid zu Cyclohexanon und Cyclohexanol Katalysatoren zur Verfügung zu stellen, die eine hohe Ausbeute und hohen Umsatz ermöglichen und zudem eine lange Lebensdauer haben sowie wenig empfindlich gegenüber Wasser sind.

Diese Aufgabe wird gelöst durch Trägerkatalysatoren mit einem Gehalt von 2 bis 30 Gew.% Kobalt in oxidischer Form, berechnet als Metall, gekennzeichnet durch A-, X- oder Y-Zeolithe als Träger, die einen Gehalt von 3–16 Gew.% Natrium in gebundener Form, berechnet als Metall, aufweisen.

Ferner ist ein Gegenstand der Erfindung die Herstellung von vorgenannten Trägerkatalysatoren, wobei man A-, X- oder Y-Zeolithe in Wasser suspendiert und unter Aufrechterhaltung eines pH-Wertes von 8 bis 11 Kobaltsalzlösungen bei einer Temperatur von 40 bis 100 °C zugibt, von der wässrigen Lösung abtrennt, trocknet und bei einer Temperatur von 200 bis 600 °C calciniert.

Ferner ist ein Gegenstand der Erfindung die Verwendung der obengenannten Kobalt enthaltenden Trägerkatalysatoren zur Herstellung von Cyclohexanol und Cyclohexanon aus Cyclohexylhydroperoxid.

Die neuen Katalysatoren haben den Vorteil, dass die Umwandlung von Cyclohexylhydroperoxid zu Cyclohexanol und zu Cyclohexanon mit hohen Ausbeuten und Umsätzen verläuft. Zudem haben die neuen Katalysatoren den Vorteil, dass sie eine lange Lebensdauer haben, wenig empfindlich gegenüber Wasser sind und das aktive Katalysatormetall nur in geringem Masse eluiert wird.

Die erfindungsgemässen Katalysatoren enthalten von 2 bis 30 Gew.% Kobalt in oxidischer Form, berechnet als Kobalt. Die Zahlenangaben beziehen sich jeweils auf die gesamte Masse aus Träger und dem katalytisch aktiven Metall. Bevorzugte Katalysatoren haben einen Gehalt von 10 bis 25 Gew.% Kobalt in oxidischer Form, berechnet als Kobalt. Darüberhinaus besitzen die Katalysatoren einen Gehalt von 3 bis 16 Gew.% Natrium in gebundener Form, berechnet als Natrium. Das katalytisch aktive Kobalt ist auf A-, X- oder Y-Zeolithe als Träger aufgebracht. Vorzugsweise ist das katalytisch aktive Kobalt in die Zeolithstruktur eingebaut. Um bei der Katalysatorherstellung eine bessere Verformbarkeit der Katalysatormasse zu erzielen, hat es sich auch bewährt, vor der Verformung z.B. bis zu 35 Gew.% aktives Aluminiumoxid oder -hydroxid, z.B. Boehmit, als Bindemittel zuzusetzen. In diesem Fall beziehen sich die obigen Zahlenangaben auf die gesamte Masse aus katalytisch aktiven Metallen, Zeolithen und Bindemitteln.

Geeignete Katalysatoren erhält man beispielsweise, indem man auf Zeolith Kobaltverbindungen, die beim Erhitzen in ihre Oxide übergehen, aus wässriger Lösung fällt. Vorteilhaft geht man von wässrigen Lösungen von Kobaltsalzen, wie Kobaltsulfat, Kobaltnitrat oder Kobaltacetat aus. Man führt die Fällung durch Zugabe von Alkalihydroxid oder -Carbonatlösung durch. Beim Fällen wird ein pH-Wert von 8 bis 11 und eine Temperatur von 40 bis 100 °C eingehalten. Der so erhaltene Kobalthydroxid und/oder Kobaltcarbonat enthaltende Zeolithträger wird anschliessend vorteilhaft mit Wasser ausgewaschen. Zweckmässig wäscht man mit alkalisiertem Wasser z.B. einer 0,1 bis 0,5 gew.%igen Natriumhydroxidlösung, damit ein bestimmter Natriumgehalt im fertigen Katalysator erreicht wird. Der so erhaltene Trägerkatalysator wird dann vorteilhaft bei 100 bis 150 °C z.B. für eine Zeit von 1 bis 20 Stunden getrocknet und dann bei einer Temperatur von 200 bis 600 °C für einen Zeitraum von z.B. 2 bis 8 Stunden calciniert. Das Calcinieren erfolgt in der Regel in Gegenwart von molekularem Sauerstoff enthaltenden Gasen, z.B. Luft.

Sollen die Katalysatoren zu Presslingen verarbeitet werden, so hat es sich bewährt, vor dem Kalzinieren z.B. bis zu 35 Gew.% aktives Aluminiumoxid oder -hydroxid, z.B. Boehmit, als Bindemittel zuzugeben und das so erhaltene Gemisch zu Presslingen zu verarbeiten. Die so erhaltenen Presslinge werden dann wie oben beschrieben gesintert.

Es wird angenommen, dass Kobalt in Form seines Oxids vorliegt, obwohl ein röntgenographischer Nachweis hierfür nicht immer möglich ist. Es ist auch ein teilweiser Ionenaustausch mit den Zeolithen während der Herstellung nicht auszuschliessen.

Neben der oben ausgeführten, bevorzugten Herstellungsweise der verwendeten Katalysatoren ist es auch möglich, die Metallverbindungen durch Tränken oder Sprühen aufzubringen und anschliessend durch Erhitzen die fertigen Katalysatoren herzustellen.

Die erfindungsgemässen Katalysatoren werden zur Herstellung von Cyclohexanon und Cyclohexanol aus Cyclohexylhydroperoxid verwendet. Vorteilhaft geht man von Lösungen von Cyclohexylhydroperoxid in Cyclohexan aus. Geeignete Lösungen enthalten beispielsweise 0,5 bis 10 Gew.% Cyclohexylhydroperoxid. Besonders bevorzugt geht man von Gemischen aus, die durch Oxidation von Cyclohexan mit molekularem Sauerstoff oder solchen enthaltenden Gasen, z.B. Luft, in flüssiger Phase bei Temperaturen von 130 bis 200°C und unter Drücken von 5 bis 25 bar, gegebenenfalls unter Mitverwendung von Katalysatoren, erhalten wurden. Vorteilhaft werden die so erhaltenen Reaktionsgemische vor der Weiterbehandlung mit Wasser, Alkalicarbonatlösungen oder Alkalilaugen gewaschen. Typische Reaktionsgemische enthalten neben Cyclohexan 1 bis 7 Gew.% Cyclohexanon und Cyclohexanol sowie 0,5 bis 5 Gew.% Cyclohexylperoxid, ferner Nebenprodukte wie Ester. Daneben können die Reaktionsgemische Wasser, z.B. bis zu 2 Gew.%, enthalten. Geeignete Reaktionsgemische erhält man beispielsweise nach dem in der DE-PS-10 46 610 beschriebenen Verfahren.

Die Behandlung des Cyclohexylhydroperoxids oder solches enthaltenden Lösungen mit den erfindungsgemässen Katalysatoren erfolgt vorteilhaft bei einer Temperatur von 40 bis 160°C, insbesondere 80 bis 120°C. Die Behandlung kann bei Atmosphärendruck oder schwach erhöhtem Druck, z.B. wie er bei der Oxidation von Cyclohexan angewandt wird, z.B. bis zu 20 bar, erfolgen. Vorteilhaft erfolgt die Behandlung von Cyclohexylhydroperoxid enthaltenden Lösungen und Reaktionsgemischen kontinuierlich, indem man solche Lösungen oder Gemische über ein fest angeordnetes Katalysatorbett leitet. Falls nach längerer Betriebszeit die Aktivität der Katalysatoren nachlässt, so lassen sich diese leicht durch Behandeln mit molekularem Sauerstoff enthaltenden Gasen, insbesondere Luft, z.B. bei einer Temperatur von 150 bis 500°C reaktivieren.

Der erfindungsgemässe Gegenstand sei an folgenden Beispielen veranschaulicht.

Beispiel 1
a) Herstellung des Katalysators
1455 g Co(NO₃)₂·6H₂O werden in 1000 g destilliertem Wasser gelöst. Diese Lösung wird zu 500 g Zeolith 4A gegeben. Am Rotationsverdampfer wird im Wasserstrahlvakuum bei ca. 95 bis 100°C Wasserbadtemperatur die wässrige Phase abgedampft, bis der Katalysator trocken ist. Der Katalysator wird zerrieben und 3 Stunden calciniert. Der Katalysator enthält dann 26,6 Gew.% Co und 7,8 Gew.% Na, eine wässrige Aufschlämmung des Kontaktes hat einen pH von 9,8 (Katalysator I).

Zur Herstellung von Strangpresslingen wird das Katalysatorpulver mit Böhmit (AlO(OH)) vermengt, ohne Peptisierung eine ½ Stunde geknetet und zu 4 mm Strangpresslingen verstrangt. Die Strangpresslinge werden dann nochmals 3 Stunden bei 300°C calciniert. Der fertige Katalysator (II) hat folgende Zusammensetzung: 8,0 Gew.% Co, 5,3 Gew.% Na, 23,2 Gew.% Al, Rest Si und O, NO₃-Gehalt 0,5%.

b) Zersetzung von Cyclohexylhydroperoxid
In einem Rührkolben, versehen mit Innenthermometer und Rückflusskühler, werden 2,0 g des o.g. Katalysatorpulvers (I) mit 100 g Oxidationsgemisch, erhalten durch Luftoxidation von Cyclohexan bei 145°C und 12 bar (Zusammensetzung: 1,81% Cyclohexanon, 2,61% Cyclohexanol, 1,48% Cyclohexylhydroperoxid, Rest Cyclohexan und Nebenprodukte wie Carbonsäuren und Carbonsäureester), bei 80°C für 30 min gerührt. Die gaschromatographische Analyse des Reaktionsproduktes hat folgende Zusammensetzung: Cyclohexanon 2,31%, Cyclohexanol 3,15%, Cyclohexylhydroperoxid 0,38%. Bezogen auf den Peroxidumsatz von 74% beträgt die Ausbeute an Anon + Anol 119%.

Vergleichsbeispiel 1
728 g Co(NO₃)₂·6H₂O werden in 500 ml destilliertem Wasser gelöst. Zu dieser Lösung werden 500 ml Aktivkohlestränge (4 mm) gegeben. Die überstehende Lösung wird abdestilliert und der Katalysator bei 100°C im Trockenschrank getrocknet. Der Tränkvorgang wird 5 mal wiederholt; dann ist die gesamte Co-Menge aufgetragen. Nach Trocknung bei 100 bis 110°C werden die Stränge bei 300°C unter einer N₂-Atmosphäre im Muffelofen calciniert. Der fertige Katalysator enthält 32,6 Gew.% Co. 2,0 g des Katalysators werden gemahlen und mit 100 g des in Beispiel 1b beschriebenen Oxidationsgemisches für 30 min bei 80°C gerührt. Der Peroxidumsatz beträgt 94%, die Ausbeute an Cyclohexanon und Cyclohexanol nur 87%.

Beispiel 2
In einem Glasrohr (Innendurchmesser 30 mm, Länge 400 m), versehen mit Doppelmantel und innenliegendem Thermoelement, werden 200 ml (150 g) des Katalysators II eingefüllt. Bei einer Temperatur von 80°C, die durch die im Mantel zirkulierende Heizflüssigkeit gehalten wird, erfolgt die Anströmung des Katalysators von unten. Der Zulauf wird mit einer Pumpe konstant gehalten. Das Zulaufgemisch stammt aus der Oxidation von Cyclohexan mit Luft und enthält neben Cyclohexan 1,64 Gew.% Cyclohexanon, 2,40 Gew.%

Cyclohexanol, 1,47 Gew.% Cyclohexylhydroperoxid, andere Peroxide 0,06 Gew.%, Säuren und Carbonsäureester (0,9 mäq/l). Zu verschiedenen Zeiten wird der Reaktionsaustrag analysiert. Die effektive Verweilzeit, bezogen auf das Lückenvolumen, beträgt 18,5 min.

| Laufzeit (h) | Peroxid-* Umsatz (%) | Cyclohexyl-** hydroperoxid Umsatz (%) | Ausbeute (%) bezogen auf Umsatz |
|---|---|---|---|
| 32 | 91 | 100 | 129 |
| 127 | 90 | 100 | 121 |
| 215 | 90 | 100 | 119 |
| 295 | 90 | 100 | 121 |
| 335 | 90 | 100 | 121 |
| 402 | 90 | 100 | 119 |
| 508 | 90 | 99 | 118 |

Nach einer Laufzeit von 508 Stunden ist der Katalysator völlig unverändert, ohne Verkrustung oder wesentlichen Aktivitätsverlust.

Vergleichsbeispiel 2

200 ml (110 g) des Co-Kohlekatalysators aus Vergleichsbeispiel 1 werden in die Versuchsapparatur gemäss Beispiel 2 eingefüllt und Oxidationsgemisch wie in Beispiel 2 beschrieben behandelt. Man erhält folgende Werte:

| Laufzeit (h) | Peroxid-* Umsatz (%) | Cyclohexyl-** hydroperoxid Umsatz (%) | Ausbeute (%) bezogen auf Umsatz |
|---|---|---|---|
| 24 | 98 | 100 | 102 |
| 56 | 96 | 100 | 100 |
| 102 | 91 | 98 | 98 |
| 184 | 85 | 97 | 93 |

\* Umsatz bez. auf die im Produktgemisch enthaltenen Peroxide
\*\* Umsatz bez. auf Cyclohexylhydroperoxid im Produktgemisch

Beispiel 3

a) Herstellung des Katalysators

Es werden 50 g Zeolith A in 2 l Wasser suspendiert, auf 80 °C erhitzt und der pH-Wert mit Natronlauge auf 10,5 bis 11 eingestellt. Zu dieser Suspension gibt man eine Lösung von 195 g Co(NO$_3$)$_2$ · 6H$_2$O in 300 ml Wasser. Die Zugabe erfolgt im Verlauf von 1 bis 2 h, wobei man durch gleichzeitige Zugabe einer Lösung von 35 g Natriumhydroxid in 300 ml Wasser für die Aufrechterhaltung des vorgenannten pH-Wertes sorgt. Nach beendeter Zugabe wird die Temperatur auf 100 °C erhöht und nach Abkühlen auf 60 bis 70 °C der Niederschlag abfiltriert und mit 1500 ml Wasser gewaschen, dessen pH-Wert durch Zugabe von Natronlauge auf etwa 8 gebracht worden ist. Anschliessend wird der Filterkuchen 18 Stunden bei 120 °C getrocknet und dann 3 h auf 300 °C erhitzt. Der so erhaltene Katalysator enthält 24,5 Gew.% Cobalt, 6,8 Gew.% Natrium sowie 0,5% Nitrat.

b) Zersetzung von Cyclohexylhydroperoxid

In einem Autoklaven werden 100 g einer Lösung von 2,0 Gew.% Cyclohexylhydroperoxid und 2,0 Gew.% Cyclohexanon in Cyclohexan zusammen mit 2,0 g des oben beschriebenen Katalysators für 30 min unter Rühren auf 80 °C erhitzt. Anschliessend wird der Katalysator abfiltriert, mit Cyclohexan gewaschen und wiederholt verwendet. Das jeweils erhaltene Filtrat wird auf Cyclohexanon, Cyclohexanol und Cyclohexylhydroperoxid analysiert. Die Ergebnisse sind nachfolgend zusammengestellt. Nach dem dritten Gebrauch sind im Reaktionsprodukt 0,03 ppm Cobalt nachweisbar.

| Versuch | Peroxidumsatz (%) | Ausbeute (%) (bez. auf Umsatz) |
|---|---|---|
| 1 | 68 | 118 |
| 2 | 68 | 117 |
| 3 | 68 | 118 |

**Patentansprüche**

1. Trägerkatalysatoren mit einem Gehalt von 2 bis 30 Gew.% Kobalt in oxidischer Form, berechnet als Kobalt, gekennzeichnet durch A-, X- oder Y-Zeolithe als Träger, die einen Gehalt von 3 bis 16 Gew.% an Natrium in gebundener Form, berechnet als Metall, haben.

2. Verfahren zur Herstellung von Trägerkatalysatoren gemäss Anspruch 1, dadurch gekennzeichnet, dass man A-, X- oder Y-Zeolithe in Wasser suspendiert und unter Aufrechterhaltung eines pH-Wertes von 8 bis 11 Kobaltsalzlösungen bei einer Temperatur von 40 bis 100 °C zugibt, von der wässrigen Lösung abtrennt, trocknet und bei 200 bis 600 °C calciniert.

3. Verwendung von Trägerkatalysatoren nach den Ansprüchen 1 und 2 zur Herstellung von Cyclohexanol und Cyclohexanon aus Cyclohexylhydroperoxid.

**Claims**

1. A supported catalyst containing from 2 to 30% by weight of cobalt in oxidic form, calculated as cobalt, wherein the carrier is an A, X or Y zeolite which contains from 3 to 16% by weight of sodium in bound form, calculated as the metal.

2. A process for the preparation of a supported catalyst as claimed in claim 1, wherein an A, X or Y zeolite is suspended in water, a cobalt salt solution is added at from 40 to 100 °C while maintaining a pH of from 8 to 11, and the product is isolated from the aqueous solution, dried, and calcined at from 200 to 600 °C.

3. The use of a supported catalyst as claimed in claims 1 and 2 for the production of cyclohexanol and cyclohexanone from cyclohexyl hydroperoxide.

**Revendications**

1. Catalyseurs sur support d'une teneur de 2 à 30% en poids en cobalt sous forme oxydée, calculée en tant que cobalt, caractérisés par des zéolithes A, X ou Y, comme support, qui ont une teneur de 3 à 16% en poids en sodium, sous forme liée, calculée en tant que métal.

2. Procédé de préparation de catalyseurs sur support selon la revendication 1, caractérisé par le fait que l'on suspend des zéolithes A, X ou Y dans l'eau et, en maintenant un pH de 8 à 11, on ajoute, à une température de 40 à 100°C, des solutions de sel de cobalt, on sépare de la solution aqueuse, on sèche et calcine entre 200 et 600°C.

3. Utilisation de catalyseurs sur support selon les revendications 1 et 2 pour la préparation de cyclohexanol, et cyclohexanone à partir d'hydro-peroxyde de cyclohexyle.